# EUROPÄISCHE PATENTSCHRIFT

(11) **EP 1 097 090 B1**
(45) Veröffentlichungstag und Bekanntmachung des Hinweises auf die Patenterteilung: **07.05.2003**
(21) Anmeldenummer: 99934662.0
(22) Anmeldetag: 12.07.1999
(51) Int. Cl.: B65D 75/58

(54) **VORRICHTUNG ZUR VERBESSERTEN ENTNAHMEFÄHIGKEIT VON BEUTELINHALTEN**
DEVICE FOR IMPROVING THE REMOVABILITY OF POUCH CONTENTS
PROCEDE POUR AMELIORER L'APTITUDE AU PRELEVEMENT DE CONTENUS DE SACHETS

(30) Priorität: 10.07.1998 DE 19830898
(43) Veröffentlichungstag der Anmeldung: 09.05.2001
(73) Patentinhaber: HEXAL AG, D-83607 Holzkirchen (DE)
(72) Erfinder: KIBELE, Ralf, D-83607 Holzkirchen (DE); KLOKKERS, Karin, D-83607 Holzkirchen (DE); SCHLUETER, Hans-Jürgen, D-83607 Holzkirchen (DE)
(86) Internationale Anmeldenummer: EP9904872
(87) Internationale Veröffentlichungsnummer: WO00002794

(56) Entgegenhaltungen:
- EP-A- 0 405 393
- EP-A- 0 635 262
- WO-A-93/23310
- WO-A-96/19394

## Beschreibung

Die Erfindung betrifft eine Vorrichtung zur Verbesserung der Entnahmefähigkeit von in Beuteln verpackten, haftklebend ausgerüsteten, flächigen Substratabschnitten auf Trägerfolien.

Die Herstellung und Verwendung flächiger haftklebender Substratabschnitte ist bekannt. Üblicherweise werden die haftklebenden Substratabschnitte auf einer Trägerschicht aufgebracht. Zum Schutz der Haftklebeschicht wird diese mit einer Abdeckfolie versehen. Die Trägerschicht kann die haftklebende Fläche zum Zwecke der Abzieherleichterung überragen. Das so zusammengesetzte System wird in einen allseitig umschließenden Beutel eingesiegelt. Unter einem Substratabschnitt wird hier ein System verstanden, welches aus einer den Wirkstoff enthaltenden Haftklebeschicht zusammen mit einer auf der einen Seite der Haftklebeschicht aufgebrachten Abdeckfolie besteht.
Der Nachteil dieser Konstruktion liegt aber darin, daß aus diesen so verpackten Substratabschnitten durch den eventuell auftretenden kalten Fluß des Haftklebers um den Substratabschnitt herum Haftkleber austreten kann, der eine Verklebung mit den Verpackungsflächen verursacht, so daß die Entnahme des Systems bei nur einseitig geöffneter Packung erschwert oder sogar unmöglich wird.

EP 635 262 A beschreibt ein Verpackungssystem für Pflaster mit wirkstoffhaltiger Klebeschicht, indem die Beutelinnenseite, Trägerschicht und/oder Abziehfolie mit Noppen versehen ist. Dadurch kann ein Ankleben des Pflasters an der Verpackung verhindert werden.

Es ist die Aufgabe der vorliegenden Erfindung, eine einfache Vorrichtung zur Verbesserung der Entnahmefähigkeit von in Beuteln verpackten, haftklebend ausgerüsteten, flächigen Substratabschnitten zu schaffen, deren Haftklebefläche auf eine Trägerschicht aufgebracht ist, welche den Substratabschnitt überragen kann. Durch die Erfindung soll insbesondere verhindert werden, daß es durch eine Wanderung des Haftklebers aufgrund von kaltem Fluß zu einer Verklebung des Systems mit der Verpackung kommt. Eine erleichterte Entnahme aus der Verpackung wird somit gewährleistet.

Es wurde nun überraschenderweise gefunden, daß durch das Einbringen von ein oder zwei einseitig trennbeschichteten Einschubkörpern ohne Erhebungen in den Beutel eine verbesserte Entnahmefähigkeit erreicht wird. Der Substratabschnitt liegt dabei auf der trennbeschichteten Seite eines Einschubkörpers auf, so daß bei eventuell auftretendem kalten Fluß des Haftklebers keine Verklebungen mit der Verpackung auftreten.

Eine weitere Ausführungsform der Erfindung besteht darin, daß sich ein oder zwei Einschubkörper, die mit Erhebungen, welche entweder durch Prägen oder Tiefziehen aus diesen selbst geformt oder auf diesen fixiert werden können, versehen sind, in dem Beutel befinden. Die Einschubkörper können dabei entweder einseitig trennbeschichtet sein oder keine Beschichtung aufweisen.

Die beiden oben beschriebenen Ausführungsformen ermöglichen zudem durch die Verwendung von den vorher beschriebenen Einschubkörpern die Verpackung mehrerer Systeme, bestehend aus einem haftklebenden Substratabschnitt und einer Trägerschicht, in einem Beutel unter Beibehaltung der verbesserten Entnahmefähigkeit.

Die Erfindung wird anhand der folgenden Figuren beispielhaft weiter erläutert.
Figur 1 zeigt die auf einer Seite des Einschubkörpers (4) fixierte trennbeschichtete Folie (6).
Figur 2 zeigt fixierte Erhebungen (5) auf dem Einschubkörper (4) im Beutel (3), die dem Substratabschnitt (2), der auf der Trägerfolie (1) befestigt ist, gegenüberliegen.
Figur 3 zeigt einen Einschubkörper (4) mit umgeknickten Ecken, welcher dem Substratabschnitt (2), der auf der Trägerfolie (1) befestigt ist, gegenüberliegt.
Figur 4 zeigt tiefgezogene oder geprägte Erhebungen (7) auf dem Einschubkörper (4).

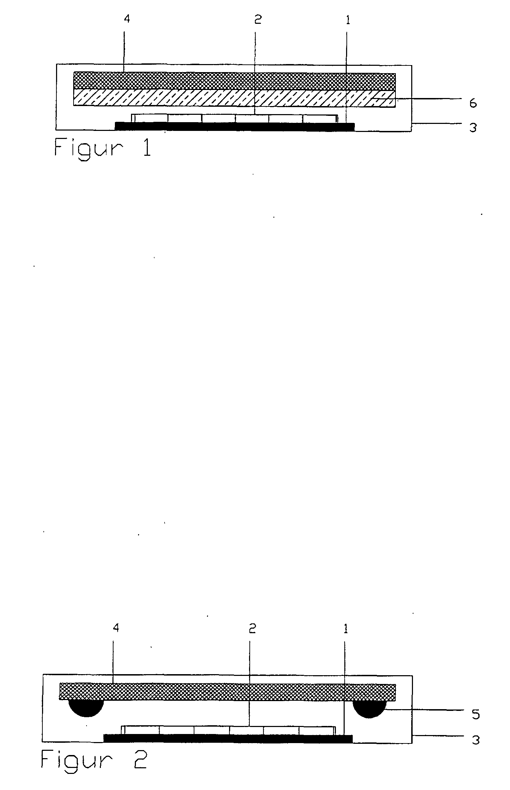

Die Trennbeschichtung (6) eines Einschubkörpers (4) (vgl. Fig. 1) kann entweder durch eine direkte Trennbeschichtung oder durch das Aufschweißen oder Aufkleben einer heißsiegelfähigen oder verklebbaren und/ oder tiefziehfähigen und/ oder prägefähigen einseitig trennbeschichteten Folie erreicht werden, wobei man als Folie Polyethylen-, Polypropylen-, Polyvinylchlorid-, Polyethylenterephthalat- und Ethylenvinylacetat-Folien sowie entsprechende Verbundfolien verwenden kann.

Die Trennbeschichtung kann z.B. durch Silikonisierung, Fluorosilikonisierung oder Teflonbeschichtung erhalten werden.

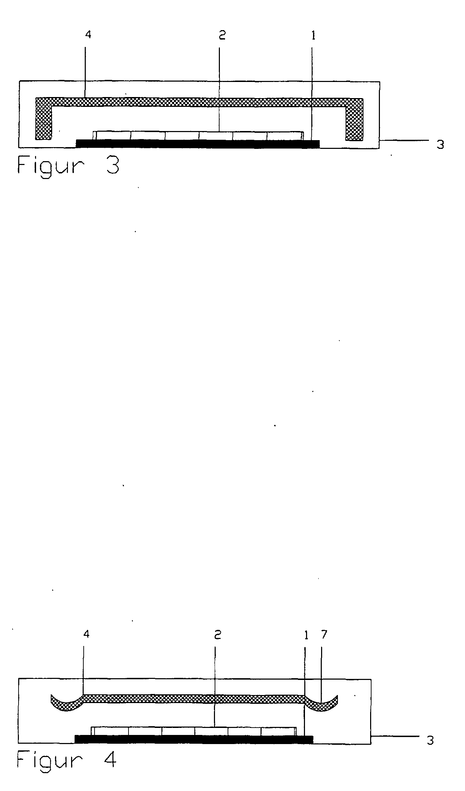

Die Erhebungen können durch zwei prinzipielle Möglichkeiten erhalten werden. Zum einen können die Erhebungen durch Hinzufügen von Fremdmaterialien oder zum anderen durch Ausnutzen des Einschubkörpers selbst erzeugt werden.
Im ersten Fall werden die Erhebungen (5) durch Aufbringen von Heiß-Schmelz-Polymeren gebildet, die nach dem Abkühlen fest auf einem oder beiden Einschubkörpern (4) verankert sind und die bei Zimmertemperatur keine Klebrigkeit besitzen. Es können auch nicht klebende Materialien durch Permanentkleber auf einem oder beiden Einschubkörpern fixiert werden. Ein Beispiel hierfür zeigt Fig. 2.
Im zweiten Falle werden durch Knick-, Präge- oder Tiefziehvorgänge die Randbereiche so verformt, daß Erhebungen entstehen. Dies kann bspw. durch Umknicken der Ecken (s. Fig. 3) oder auch durch Knicken von durch Einschneiden der Seiten des Einschubkörpers erhaltenen Laschen erreicht werden. Eine weitere Möglichkeit bietet das Tiefziehen oder Prägen von Noppen (7) im Einschubkörper (4), wie in Fig. 4 dargestellt wird.
Auch Stege, die aus dem Einschubkörper geprägt oder tiefgezogen sind und die Ecken des Substratabschnittes umwinkeln oder zusammenhängend parallel zu den Rändern des Substratabschnittes verlaufen, dienen als Erhebungen, die die verbesserte Entnahmefähigkeit gewährleisten. Die Erhebungen können dabei das System, bestehend aus einem haftklebenden Substratabschnitt und einer Trägerschicht, überragen.
Als Material für den Einschubkörper eignen sich heißsiegelfähige und/ oder tiefziehfähige und/ oder prägefähige Folien oder Folienverbunde mit eventuellem Aluminiumanteil.
Die erfindungsgemäße Vorrichtung wird besonders zur Verpackung von Pflastern, transdermalen therapeutischen Systemen oder dergleichen angewendet.

## Patentansprüche

1. Vorrichtung zur Verbesserung der Entnahmefähigkeit von in Beuteln (3) verpackten, haftklebend ausgerüsteten flächigen Substratabschnitten (2), deren Haftklebefläche auf eine Trägerschicht (1) aufgebracht ist, welche den Substratabschnitt überragen kann, **dadurch gekennzeichnet, daß** sich ein oder zwei einseitig trennbeschichtete (6) Einschubkörper (4) ohne Erhebungen in dem Beutel befinden, wobei das substrathaltige System mit dem Substratabschnitt auf einem trennbeschichteten Einschubkörper aufliegt.

2. Vorrichtung zur Verbesserung der Entnahmefähigkeit von in Beuteln (3) verpackten, haftklebend ausgerüsteten flächigen Substratabschnitten (2), deren Haftklebefläche auf eine Trägerschicht (1) aufgebracht ist, welche den Substratabschnitt überragen kann, **dadurch gekennzeichnet, daß** sich ein oder zwei Einschubkörper (4), die mit Erhebungen, welche entweder durch Prägen oder Tiefziehen aus diesen selbst geformt oder auf diesen fixiert werden, in dem Beutel befinden, wobei die Einschubkörper trennbeschichtet sein können.

3. Vorrichtung nach Anspruch 1 und 2, **dadurch gekennzeichnet, daß** die Trennbeschichtung der Einschubkörper entweder durch eine direkte Trennbeschichtung oder durch das Aufschweißen oder Aufkleben einer einseitig trennbeschichteten Folie erreicht wird.

4. Vorrichtung nach einem der vorangegangenen Ansprüche, **dadurch gekennzeichnet, daß** die Folie heißsiegelfähig oder verklebbar und/ oder tiefziehfähig und/ oder prägefähig ist, insbesondere handelt es sich um Polyethylen-, Polypropylen-, Polyvinylchlorid- Polyethylenterephthalat- und Ethylenvinylacetat-Folien sowie entsprechende Verbundfolien.

5. Vorrichtung nach einem der vorangegangenen Ansprüche, **dadurch gekennzeichnet, daß** die Trennbeschichtung in Form einer Silikonisierung, Fluorosilikonisierung oder Teflonbeschichtung erhalten wird.

6. Vorrichtung nach einem der vorhergehenden Ansprüche, **dadurch gekennzeichnet, daß** sich mehrere Systeme, bestehend aus einem haftklebenden Substratabschnitt und einer Trägerschicht, in einem Beutel, getrennt durch jeweils einen entsprechenden Einschubkörper, befinden.

7. Vorrichtung nach einem der vorangegangenen Ansprüche, **dadurch gekennzeichnet, daß** die Erhebungen aus Heiß-Schmelz-Polymeren bestehen, die bei Zimmertemperatur keine Klebrigkeit besitzen.

8. Vorrichtung nach einem der vorangegangenen Ansprüche, **dadurch gekennzeichnet, daß** die Erhebungen aus nicht klebenden Materialien gebildet sind, die mit Permanentklebern auf dem (den) Einschubkörper(n) fixiert sind.

9. Vorrichtung nach einem der vorangegangenen Ansprüche, **dadurch gekennzeichnet, daß** die Erhebungen durch Umknicken der Ecken der Einschubkörper gebildet werden.

10. Vorrichtung nach einem der vorangegangenen Ansprüche, **dadurch gekennzeichnet, daß** die Erhebungen durch Umknicken von durch Einschneiden der Ränder der Einschubkörper erhaltene Laschen gebildet werden.

11. Vorrichtung nach einem der vorangegangenen Ansprüche, **dadurch gekennzeichnet, daß** die Erhebungen durch Prägung oder Tiefziehen von Noppen in den Einschubkörpern gebildet werden.

12. Vorrichtung nach einem der vorangegangenen Ansprüche, **dadurch gekennzeichnet, daß** die Erhebungen durch Prägen oder Tiefziehen die Ecken des Substratabschnitts umwinkelnde Stege in den Einschubkörpern gebildet werden.

13. Vorrichtung nach einem der vorangegangenen Ansprüche, **dadurch gekennzeichnet, daß** die Erhebungen durch Prägung oder Tiefziehen von zusammenhängenden Stegen parallel zu den Rändern des Substratabschnittes in den Einschubkörpern gebildet werden.

14. Verwendung der Vorrichtung nach einem der vorangegangenen Ansprüche zur Verpackung von Pflastern, transdermalen therapeutischen Systemen oder dergleichen.

## Claims

1. Device for improving the removability of flat substrate sections (2) being packaged in pouches (3) and rendered pressure-sensitive adhesive, said pressure-sensitive adhesive layer being fixed on a supporting layer (1), which can overlap said substrate section wherein one or two inserts (4) being nonstick coated (6) on one side without any evaluations are located in the pouch, whereby the substrate section of the substrate containing system is laying on a nonstick coated insert.

2. Device for improving the removability of flat substrate sections (2) being packaged in pouches (3) and rendered pressure-sensitive adhesive, the pressure-sensitive adhesive layer being fixed on a supporting layer (1), which can overlap said substrate section wherein one or two inserts (4) are located in the pouch, said inserts having evaluations either formed by embossing or deep-drawing from the insert itself or fixed on the insert, whereby said evaluations could be nonstick coated.

3. Device according to claims 1 and 2 wherein said nonstick coating of the inserts is obtained either by a direct nonstick coating or by welding or sicking of a foil, said foil being nonstick coated on one side.

4. Device according to any one of the preceding claims wherein said foil can be used for heat-sealing or sicking and/or deep-drawing and/or embossing, in particular foils like polyethylene, polypropylene, polyvinylchlorid, polyethylene-terephthalate and ethylenevinyl acetate foils as well as composite foils.

5. Device according to any one of the preceding claims wherein said nonstick coating is obtained by siliconisation, fluoro-siliconisation or teflon coating.

6. Device according to any one of the preceding claims wherein several systems consisting of a pressure-sensitive adhesive substrate section and a supporting layer, are located in a pouch and these systems are separated from each other by an adequate insert.

7. Device according to any one of the preceding claims wherein said evaluations consist of hot-melt-polymers which are not tacky at room temperature.

8. Device according to any one of the preceding claims wherein said evaluations are formed from nonsticking materials which are fixed on the insert(s) by means of a permanent adhesive.

9. Device according to any one of the preceding claims wherein said evaluations are formed by folding the edges of the inserts.

10. Device according to any one of the preceding claims wherein said evaluations are formed by folding straps, said straps being obtained by cutting-in the borders of the inserts.

11. Device according to any one of the preceding claims wherein said evaluations are formed by embossing or deep-drawing knobs in the inserts.

12. Device according to any one of the preceding claims wherein said evaluations are formed by embossing or deep-drawing bars in the inserts, said bars forming angles around the edges of the substrate section.

13. Device according to any one of the preceding claims wherein said evaluations are formed by embossing or deep-drawing of coherent bars in the inserts, which run parallel to the borders of the substrate section.

14. The use of a device as defined in any one of the preceding claims for the packaging of plasters, transdermal therapeutic systems, and the like.

## Revendications

1. Dispositif pour améliorer l'aptitude au prélèvement de pièces de substrat (2) plates adhésives conditionnées dans des sachets (3), pièces dont la surface adhésive est appliquée sur une couche support (1) qui peut déborder de la pièce de substrat, **caractérisé en ce que** dans le sachet se trouvent un ou deux éléments d'insertion (4) sans saillies, enduits d'un seul côté d'un revêtement de séparation (6), le système contenant le substrat reposant par sa pièce de substrat sur un élément d'insertion enduit du revêtement de séparation.

2. Dispositif pour améliorer l'aptitude au prélèvement de pièces de substrat (2) plates adhésives conditionnées dans des sachets (3), pièces dont la surface adhésive est appliquée sur une couche support (1) qui peut déborder de la pièce de substrat, **caractérisé en ce que** dans le sachet se trouvent un ou deux éléments d'insertion (4) avec saillies, saillies qui peuvent soit être formées par estampage ou par emboutissage de l'élément d'insertion même, soit être fixées sur l'élément d'insertion, les éléments d'insertion pouvant être enduits d'un revêtement de séparation.

3. Dispositif selon la revendication 1 ou 2, **caractérisé en ce que** le revêtement de séparation des éléments d'insertion est obtenu soit par revêtement direct, soit par soudage ou collage d'une feuille enduite d'un seul côté d'un revêtement de séparation.

4. Dispositif selon l'une des revendications précédentes, **caractérisé en ce que** la feuille peut être thermoscellée ou collée et/ou emboutie et/ou estampée, il s'agit en particulier de feuilles en polyéthylène, en polypropylène, en chlorure de polyvinyle, en polytéréphtalate d'éthylène et en copolymères d'éthylène - acétate de vinyle ainsi que de feuilles stratifiées correspondantes.

5. Dispositif selon l'une des revendications précédentes, **caractérisé en ce que** le revêtement de séparation est obtenu sous la forme d'une siliconisation, d'une fluorosiliconisation ou d'un revêtement en téflon.

6. Dispositif selon l'une des revendications précédentes, **caractérisé en ce que** plusieurs systèmes, se composant d'une pièce de substrat adhésive et d'une couche support, se trouvent dans un sachet, toujours séparés par un élément d'insertion correspondant.

7. Dispositif selon l'une des revendications précédentes, **caractérisé en ce que** les saillies sont en polymères thermoplastiques qui ne présentent pas de pégosité à température ambiante.

8. Dispositif selon l'une des revendications précédentes, **caractérisé en ce que** les saillies sont formées en des matériaux non collants qui sont fixés sur l'(es) élément(s) d'insertion à l'aide de colles permanentes.

9. Dispositif selon l'une des revendications précédentes, **caractérisé en ce que** les saillies sont formées par pliage des coins des éléments d'insertion.

10. Dispositif selon l'une des revendications précédentes, **caractérisé en ce que** les saillies sont formées par pliage des languettes obtenues par découpage des bords des éléments d'insertion.

11. Dispositif selon l'une des revendications précédentes, **caractérisé en ce que** les saillies sont formées dans les éléments d'insertion par estampage ou emboutissage de nopes.

12. Dispositif selon l'une des revendications précédentes, **caractérisé en ce que** les saillies sont formées dans les éléments d'insertion par estampage ou emboutissage de traverses entourant les coins de la pièce de substrat.

13. Dispositif selon l'une des revendications précédentes, **caractérisé en ce que** les saillies sont formées dans les éléments d'insertion par estampage ou emboutissage de traverses continues parallèles aux bords de la pièce de substrat.

14. Utilisation du dispositif selon l'une des revendications précédentes pour le conditionnement de pansements, de systèmes thérapeutiques transdermiques ou similaires.
